## Europäisches Patentamt
## European Patent Office
## Office européen des brevets

(11) Numéro de publication: **0 202 165**
**B1**

(12) **FASCICULE DE BREVET EUROPEEN**

(45) Date de publication du fascicule du brevet:
**03.05.89**

(21) Numéro de dépôt: **86401007.9**

(22) Date de dépôt: **12.05.86**

(51) Int. Cl.⁴: **C07H 15/04**

(54) **Procédé et installation de production de maltitol cristallisé.**

(30) Priorité: **15.05.85 FR 8507431**

(43) Date de publication de la demande:
**20.11.86 Bulletin 86/47**

(45) Mention de la délivrance du brevet:
**03.05.89 Bulletin 89/18**

(84) Etats contractants désignés:
**CH DE FR IT LI NL**

(56) Documents cités:
**EP-A- 0 039 981**
**FR-A- 2 499 576**

(73) Titulaire: **Roquette Frères, F-62136 Lestrem(FR)**

(72) Inventeur: **Gouy, Pierre-Antoine, 59, Chemin Vert,
F-59130 Lambersart(FR)**
Inventeur: **Leleu, Jean-Bernard, 22, Place du 8 mai,
F-62136 Lestrem(FR)**

(74) Mandataire: **Koch, Gustave et al, Cabinet
PLASSERAUD 84, rue d'Amsterdam, F-75009 Paris(FR)**

EP 0 202 165 B1

## Description

L'invention a pour objet un procédé et une installation de production de maltitol cristallisé.

Il est connu par le brevet FR-A 2 499 576 de préparer le maltitol cristallisé par refroidissement de sirops riches en maltitol en présence de cristaux de maltitol qui jouent le rôle de germes de cristallisation.

Ce procédé ne donne pas entièrement satisfaction.

On connait, par ailleurs, par EP-A 0 147 269 un procédé de cristallisation comportant un recyclage de dextrose monohydrate.

Pour faire face aux contraintes notamment économiques toujours plus sévères, la Société Demanderesse a cherché à mettre au point un procédé et une installation répondant mieux que ceux qui existent déjà aux divers desiderata de la pratique, en particulier du point de vue de la productivité de l'opération de cristallisation par unité de volume de l'appareillage utilisé.

Et elle a trouvé que ce but pouvait être atteint grâce à un procédé caractérisé par le fait
— que l'on fait parcourir à une masse formée de sirop et de cristaux de maltitol, de haut en bas, en continu et sous malaxage, une zone de cristallisation de direction verticale ou inclinée, dans laquelle est établi un gradient de température globalement décroissant vers le bas éventuellement modulé de 0,2 à 2°C/heure, de préférence de 0,5 à 2 et plus préférentiellement encore de 0,5 à 1,2°C/heure de traitement,
— que l'on alimente ladite zone de cristallisation au voisinage de son extrémité supérieure, d'une part, en sirop de maltitol ayant une richesse en maltitol supérieure à 80%, de préférence de 88 à 98% et plus préférentiellement encore de 90 à 95% en poids et un taux de matières sèches supérieur à 65%, de préférence de 70 à 87% et plus préférentiellement encore de 70 à 85% en poids et, d'autre part, en masse soumise à la cristallisation qui est prélevée et recyclée à partir d'un niveau intermédiaire de la zone de cristallisation, distant de ses extrémités d'au moins 1/6, de préférence 1/5 et plus préférentiellement encore 1/3 de la longueur totale de ladite zone, la quantité de masse soumise à la cristallisation et recyclée représentant en volume de 40 à 110% et de préférence de 80 à 100% de la quantité de sirop de maltitol introduite dans la zone, et
— que l'on extrait, en continu, au voisinage de l'extrémité inférieure de la zone de cristallisation, un produit fortement enrichi en cristaux de maltitol à partir duquel on récupère lesdits cristaux.

Pour mettre en œuvre le susdit procédé, on a recours, conformément à l'invention, à une installation constituée essentiellement d'une enceinte de cristallisation d'axe vertical ou incliné et équipée
— d'un système d'alimentation en sirop riche en maltitol au voisinage de son extrémité supérieure,
— d'un système de malaxage et d'un système de régulation de température propres à établir à l'intérieur de l'enceinte et au sein de la masse soumise à la cristallisation contenue dans l'enceinte, un gradient de température globalement décroissant de haut en bas,
— d'un système d'extraction continue au voisinage de son extrémité inférieure, d'un produit fortement enrichi en cristaux de maltitol qui est acheminé par des moyens appropriés vers un système propre à récupérer les cristaux à partir de ce produit, et
— de moyens propres à prélever à un niveau intermédiaire de l'enceinte, distant des extrémités de celle-ci d'au moins 1/6, de préférence 1/5 et plus préférentiellement encore 1/3 de la longueur totale, une fraction de la masse soumise à la cristallisation et à recycler cette fraction dans l'enceinte à un niveau voisin de l'extrémité supérieure de celle-ci.

L'invention vise également d'autres dispositions qui s'utilisent de préférence en même temps et dont il sera plus explicitement question ci-après.

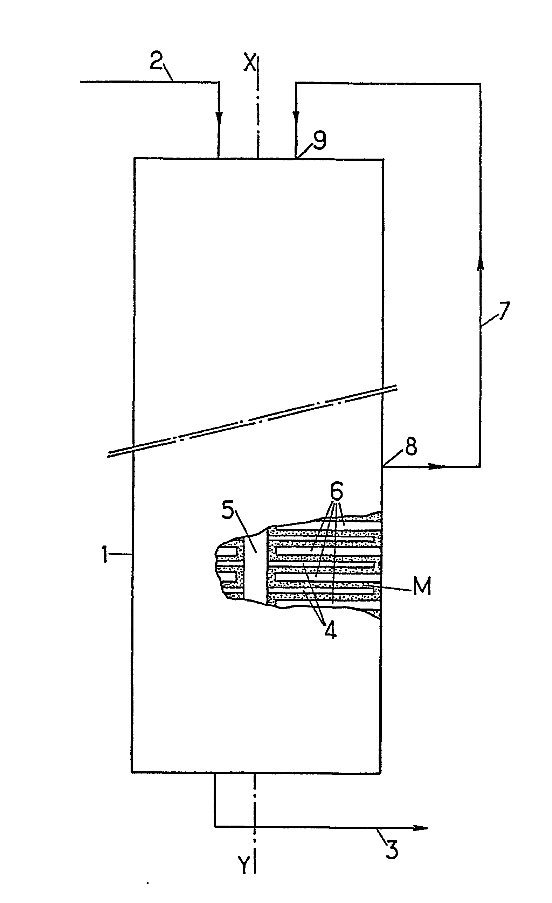

Et elle pourra, de toute façon, être bien comprise à l'aide du complément de description qui suit et du dessin annexé qui sont relatifs à des modes de réalisation avantageux.

La figure unique du dessin montre schématiquement une installation conforme à l'invention.

Se proposant, par conséquent, de produire du maltitol cristallisé conformément à l'invention, on s'y prend comme suit ou de façon équivalente.

On utilise comme matière première des sirops de maltitol provenant d'un éluat de chromatographie riche en maltitol, présentant une teneur en matières sèches supérieure à 65% et, de préférence, d'environ 70 à 87% en poids, le maltitol entrant pour au moins 80% et, de préférence, pour une proportion de 88% à 98% et plus préférentiellement encore de 90 à 95% en poids, dans la constitution sur matières sèches du sirop.

Ce sirop est, de préférence, sensiblement exempt de cristaux de maltitol et de nucléi.

Il est acheminé vers une zone de cristallisation verticale ou inclinée, qu'il parcourt en continu de haut en bas à partir d'un point situé au voisinage de son extrémité supérieure et à l'intérieur de laquelle il est soumis, en présence de cristaux de maltitol jouant le rôle de germes de cristallisation, à un malaxage et à un gradient de température globalement décroissant de haut en bas.

La température du sirop est amenée ou maintenue, au moment de son introduction dans la zone de cristallisation, à une valeur choisie dans l'intervalle de 50 à 90°C, de préférence de 55 à 80°C et plus préférentiellement de 60 à 70°C.

Le gradient de température établi à l'intérieur de la zone de cristallisation au sein de la masse soumise à la cristallisation correspond à une diminution de 0,2 à 2°C, de préférence de 0,5 à 1,2°C par heure de traitement et est tel qu'à la sortie de ladite zone, à un point situé au voisinage de l'extrémité inférieure de celle-ci, la masse soumise à cristallisation qui comprend le sirop, les cristaux initialement présents et ceux formés par le phénomène de cristallisation, se trouve amenée à une température située à l'intérieur d'un intervalle de 40 à 20°C, de préférence de 30 à 25°C.

Au fur et à mesure que la masse soumise à la cristallisation se rapproche de l'extrémité inférieure de la zone de cristallisation, sa richesse en cristaux de maltitol augmente.

L'obtention, au voisinage de l'extrémité inférieure de la zone de cristallisation d'une masse riche en cristaux qui puisse être extraite en continu sans dérèglement des paramètres du processus de cristallisation, dérèglement qui se répercuterait au niveau de l'étape suivante de séparation de la phase liquide et des cristaux et qui pourrait nécessiter des arrêts intermittents de l'installation, est rendue possible, par le procédé selon l'invention et notamment grâce au prélèvement, à un niveau médian distant des extrémités de la zone d'au moins 1/6, de préférence 1/5 et plus préférentiellement encore 1/3 de la longueur totale d'une fraction de la masse soumise à cristallisation qui est recyclée et réintroduite dans la zone de cristallisation à un niveau voisin de son extrémité supérieure.

La fraction prélevée et recyclée représente, en volume, de 40 à 110% et de préférence de 80 à 100% du volume de sirop de maltitol alimentant la zone de cristallisation.

Le débit d'alimentation en sirop de maltitol est choisi de façon telle que le temps de séjour moyen, d'une fraction donnée de la masse soumise à cristallisation à l'intérieur de la zone de cristallisation soit de 40 à 80 heures, de préférence de 48 à 65 heures; la valeur adoptée dépend des capacités d'échange thermique des moyens comportés par la zone et à l'aide desquels est établi, à l'intérieur de ladite zone au sein de la masse soumise à la cristallisation, le gradient de température décroissant.

La viscosité de la masse soumise à cristallisation qui augmente au fur et à mesure que croît la proportion de cristaux de maltitol, c'est-à-dire dans le sens descendant, fait que la zone de cristallisation est, de préférence, équipée de moyens de refoulement ou d'aspiration propres à faciliter le cheminement de la masse à l'intérieur de la zone.

Par ailleurs, les moyens de malaxage et d'homogénéisation comportés par la zone de cristallisation doivent être agencés de telle sorte que les zones mortes soient évitées et que l'échange thermique entre la masse soumise à cristallisation et les moyens de refroidissement soit le plus efficace possible.

Le produit extrait de la zone de cristallisation et qui constitue, comme déjà indiqué, une masse riche en cristaux de maltitol, comprend des cristaux de maltitol d'un spectre granulométrique caractérisé par une faible proportion de fins et de gros cristaux et par une forte proportion de cristaux de taille intermédiaire, ce spectre ne variant pas dans le temps, ce grâce à quoi l'étape de traitement suivante, qui consiste à séparer ces cristaux de la phase liquide dans laquelle ils baignent, ne connaît pas de perturbation.

Cette séparation comprend un essorage et éventuellement un lavage grâce auxquels on récupère la majeure partie de la phase liquide; celle-ci forme des eaux-mères dont la concentration en maltitol est inférieure à celle du sirop de maltitol de départ – cette concentration atteint généralement de 70 à 96% et plus généralement encore de 80 à 90% – et dans lesquelles on retrouve la presque totalité des impuretés contenues dans le sirop de maltitol de départ.

Les eaux-mères recueillies peuvent être partiellement recyclées.

Ceci étant, pour mettre en œuvre le procédé conforme à l'invention, on peut avoir recours à une enceinte unique 1 ayant la forme d'un cylindre de révolution d'axe XY.

L'axe XY est disposé avantageusement suivant la verticale mais peut également être incliné.

L'enceinte est équipée
– d'un système d'alimentation en sirop de maltitol au niveau de l'extrémité supérieure de l'enceinte et représenté schématiquement par une canalisation 2,
– d'un système de malaxage et de régulation de la température dont il va être question et
– d'un système d'extraction en continu au niveau de l'extrémité inférieure de l'enceinte et schématiquement représenté par une canalisation 3, ce système étant propre à récupérer la masse riche en cristaux de maltitol obtenue à la sortie de la zone de cristallisation; ce système d'extraction peut comporter des moyens d'aspiration non représentés qui coopèrent à faire parcourir l'enceinte à la masse soumise à la cristallisation.

Le système de malaxage et de régulation de la température dont il est question ci-dessus peut avantageusement comporter
– un ensemble de bras de malaxage 4 portés à intervalles réguliers par un arbre rotatif 5 dont l'axe est confondu avec l'axe XY de l'enceinte,
– des nappes de refroidissement 6 disposées en alternance avec les bras malaxeurs 4 et portées par la paroi de l'enceinte 1, ces nappes de refroidissement étant parcourues par un fluide de refroidissement.

Conformément à l'invention, l'enceinte comporte en outre des moyens globalement représentés en 7 et propres
– à prélever à un niveau intermédiaire 8 de l'enceinte, distant des extrémités de celle-ci d'au moins 1/6, de préférence 1/5 et plus préférentiellement encore 1/3 de la longueur totale, une fraction de la masse M soumise à cristallisation et parcourant l'enceinte de haut en bas et
– à recycler cette fraction à un niveau 9 situé au voisinage de l'extrémité supérieure de l'enceinte.

La capacité d'échange thermique, le système de régulation de température, la vitesse de rotation des moyens de malaxage et la vitesse avec laquelle, sous l'influence des moyens d'aspiration non représentés, la masse soumise à cristallisation parcourt l'enceinte, c'est-à-dire la durée moyenne de séjour d'une fraction donnée de cette masse à l'intérieur de l'enceinte, sont choisies de façon telle que s'établisse, au sein de l'ensemble de la masse soumise à cristallisation, le gradient de température prévu conformément à l'invention.

On signale que, dans la pratique, le fluide de refroidissement est de l'eau et que l'écart moyen de température en un point donné de l'enceinte entre cette eau et la masse soumise à cristallisation, est de l'ordre de 2 à 10°C.

EXEMPLE

a) On a recours à une installation conforme à l'invention comportant une enceinte cylindrique unique d'un volume utile de 3 m³.

On introduit dans cette enceinte, avec un débit de 50 l/h, un sirop de maltitol ayant une teneur en matières sèches de 82,6% et comprenant 92% en poids sur matières sèches de maltitol, les 8% restants étant constitués par du sorbitol, du maltotriitol et des oligosaccharides hydrogénés.

La température du sirop à l'entrée de l'enceinte est d'environ 65°C.

Simultanément on recycle, avec un débit de 40 l par heure, une fraction de la masse en cours de cristallisation prélevée à un niveau sensiblement médian de l'enceinte.

La durée de passage moyen à l'intérieur de l'enceinte d'une fraction donnée de la masse soumise à la cristallisation est d'environ 60 heures.

La masse riche en cristaux extraite au niveau de l'extrémité inférieure de l'enceinte se trouve à une température voisine de 30°C, le gradient de température globalement décroissant de haut en bas correspondant donc à environ 0,6°C par heure.

La teneur en maltitol des eaux-mères récupérées après séparation et lavage des cristaux de maltitol est de 85% sur matières sèches, le complément à 100 étant constitué par du sorbitol, du maltotriitol et des oligosaccharides hydrogénés.

Le rendement de cristallisation qui est donné par la formule:

$$r = \frac{A - H}{100 - H}$$

dans laquelle
- A, qui représente la richesse en maltitol du sirop d'alimentation, est égale à 92% et
- H, qui représente la richesse des eaux-mères, est de 85% après lavage des cristaux,
s'établit à 46,7%.

On produit par jour 640 kg de maltitol, ce qui correspond à une productivité de 0,213 tonne par jour et par m³ de l'enceinte.

Il ne se produit aucune perturbation nécessitant l'arrêt de l'installation qui fonctionne en continu.

Les cristaux recueillis après essorage et lavage présentent d'excellentes propriétés physiques et chimiques.

Ces cristaux sont d'une pureté supérieure à 99%, leur indice d'écoulement est bon et leur répartition granulométrique est la suivante:
- cristaux de taille supérieure à 40 μm: 85%
- cristaux de taille inférieure à 250 μm: 95%.

b) La même installation et les mêmes conditions opérationnelles sont utilisées.

Cependant, à un moment donné, après avoir atteint l'équilibre du système, la fraction recyclée est prélevée à un niveau situé à l'extérieur du domaine conforme à l'invention.

On constate alors rapidement une évolution des paramètres de cristallisation qui se manifeste après quelques heures par une mauvaise séparation au niveau du turbinage et qui finit par nécessiter l'arrêt de l'installation et l'enlèvement de la masse qui la remplit avant de la remettre en marche sous les conditions conformes à l'invention.

## Revendications

1. Procédé de production de maltitol cirstallisé caractérisé par le fait
- que l'on fait parcourir à une masse formée de sirop et de cristaux de maltitol, de haut en bas, en continu et sous malaxage, une zone de cristallisation de direction verticale ou inclinée, dans laquelle est établi un gradient de température globalement décroissant vers le bas éventuellement modulé de 0,2 à 2°C/heure de traitement,
- que l'on alimente ladite zone de cristallisation au voisinage de son extrémité supérieure, d'une part, en sirop de maltitol ayant une richesse en maltitol supérieure à 80% en poids et un taux de matières sèches supérieur à 65% et, d'autre part, en masse soumise à la cristallisation qui est prélevée et recyclée à partir d'un niveau intermédiaire de la zone de cristallisation, distant de ses extrémités d'au moins 1/6, de préférence 1/5 et plus préférentiellement encore 1/3 de la longueur totale de ladite zone, la quantité de masse soumise à la cristallisation et recyclée représentant en volume de 40 à 110% de la quantité de sirop de maltitol introduite dans la zone, et
- que l'on extrait, en continu, au voisinage de l'extrémité inférieure de la zone de cristallisation, un produit fortement enrichi en cristaux de maltitol à partir duquel on récupère lesdits cristaux.

2. Procédé selon la revendication 1, caractérisé par le fait que le gradient de température globalement décroissant est de 0,5 à 1,2°C/heure de traitement.

3. Procédé selon l'une des revendications 1 et 2, caractérisé par le fait que le sirop servant de matière première présente une richesse en maltitol de 88 à 98%, de préférence de 90 à 95% en poids et un taux de matières sèches de 70 à 87% en poids.

4. Procédé selon l'une des revendications 1 à 3, caractérisé par le fait que la fraction recyclée représente en volume de 80 à 100% du volume de sirop de maltitol alimentant la zone de cristallisation.

5. Procédé selon l'une des revendications 1 à 4, caractérisé par le fait que la température du sirop de maltitol au moment de son introduction dans la zone de cristallisation est de 50 à 90°C, de préférence de 55 à 80°C et plus préférentiellement de 60 à 70°C et que la masse extraite de la zone de cristallisation se trouve à la sortie de celle-ci à une température de 40 à 20°C, de préférence de 30 à 25°C.

6. Installation de production de maltitol cristallisé caractérisée par le fait qu'elle comprend essentiellement une enceinte de cristallisation d'axe vertical ou incliné et équipée.
- d'un système d'alimentation en sirop riche en maltitol au voisinage de son extrémité supérieure.
- d'un système de malaxage et d'un système de régulation de température propres à établir à l'intérieur de l'enceinte et au sein de la masse soumise à la cristallisation contenue dans l'enceinte, un gradient de température globalement décroissant de haut en bas.
- d'un système d'extraction continue au voisinage de son extrémité inférieure, d'un produit fortement enrichi en cristaux de maltitol qui est acheminé par des moyens appropriés vers un système propre à récupérer les cristaux à partir de ce produit, et

– de moyens propres à prélever à un niveau intermédiaire de l'enceinte, distant des extrémités de celle-ci d'au moins 1/6, de préférence 1/5 et encore plus préférentiellement 1/3 de sa longueur totale, une fraction de la masse soumise à la cristallisation et à recycler cette fraction dans l'enceinte à un niveau voisin de l'extrémité supérieure de celle-ci.

**Patentansprüche**

1. Verfahren zur Herstellung von kristallisiertem Maltit, dadurch gekennzeichnet, daß man eine aus einem Sirup und aus Maltitkristallen gebildete Masse von oben nach unten kontinuierlich und unter Mischen eine Kristallisationszone mit vertikaler oder geneigter Richtung durchlaufen läßt, in der für einen gegebenenfalls modulierten insgesamt nach unten abnehmenden Temperaturgradienten von 0,2 bis 2°C/Stunde Behandlung Vorsorge getroffen ist, daß man diese Kristallisationszone in der Nähe ihres oberen Endes einesteils mit einem Maltitsirup, der einen Maltitgehalt von höher als 80 Gew.-% und einen Trockensubstanzgehalt von höher als 65% besitzt, und anderenteils mit einer der Kristallisation unterliegenden Masse beschickt, die von einem Zwischenbereich der Kristallisationszone, der von ihren Enden zumindest 1/6, vorzugsweise 1/5 und insbesondere 1/3 der Gesamtlänge dieser Zone entfernt ist, entnommen und recyclisiert wird, wobei die Menge der der Kristallisation unterliegenden und recyclisierten Masse 40 bis 110 Vol.-% der Menge des in die Zone eingeführten Maltitsirups ausmacht, und daß man kontinuierlich in der Nähe des unteren Endes der Kristallisationszone ein an Maltitkristallen stark angereichertes Produkt extrahiert, aus dem man die Kristalle gewinnt.

2. Verfahren gemäß Anspruch 1, dadurch gekennzeichnet, daß der insgesamt abnehmende Temperaturgradient 0,5 bis 1,2°C/Stunde Behandlung beträgt.

3. Verfahren gemäß einem der Ansprüche 1 und 2, dadurch gekennzeichnet, daß der als Ausgangsmaterial dienende Sirup einen Maltitgehalt von 88 bis 98 Gew.-%, vorzugsweise 90 bis 95 Gew.-%, und einen Trockensubstanzgehalt von 70 bis 87 Gew.-% besitzt.

4. Verfahren gemäß einem der Ansprüche 1–3, dadurch gekennzeichnet, daß die recyclisierte Fraktion auf Volumen bezogen 80 bis 100% des Volumens des der Kristallisationszone zugeführten Maltitsirups beträgt.

5. Verfahren gemäß einem der Ansprüche 1–4, dadurch gekennzeichnet, daß die Temperatur des Maltitsirups zum Zeitpunkt seines Einbringens in die Kristallisationszone 50 bis 90°C, vorzugsweise 55 bis 80°C und insbesondere 60 bis 70°C beträgt, und daß die extrahierte Masse der Kristallisationszone sich am Ausgang derselben bei einer Temperatur von 40 bis 20°C, vorzugsweise 30 bis 25°C, befindet.

6. Vorrichtung zur Herstellung von kristallisiertem Maltit, dadurch gekennzeichnet, daß sie im wesentlichen einen Kristallisationsbehälter mit vertikaler oder geneigter Achse umfaßt, der ausgestattet ist mit einem System für die Zufuhr eines an Maltit reichen Sirups in der Nähe seines oberen Endes, einem System zum Mischen und einem System zur Temperaturregulierung, die im Stande sind, im Inneren des Behälters und innerhalb der der Kristallisation unterzogenen Masse, die in dem Behälter enthalten ist, einen von oben nach unten insgesamt abnehmenden Temperaturgradienten zu schaffen, einem System in der Nähe seines unteren Endes für die kontinuierliche Extraktion eines stark an Maltitkristallen angereicherten Produkts, das mit Hilfe geeigneter Mittel über ein für die Gewinnung der Kristalle aus diesem Produkt geeignetes System transportiert wird und geeigneten Mitteln für die Entnahme in einem Zwischenbereich des Behälters, der von dessen Enden zumindest 1/6, vorzugsweise 1/5 und insbesondere 1/3, seiner Gesamtlänge entfernt ist, einer Fraktion der der Kristallisation unterzogenen Masse und für die Recyclisierung dieser Fraktion in den Behälter in einem Bereich in der Nähe des oberen Endes desselben.

**Claims**

1. Method of producing crystalline maltitol characterized by the fact
– that a mass formed from syrup and from crystals of maltitol is made to traverse, from top to bottom, continuously and with malaxation, a crystallization zone of vertical or inclined direction, in which there is established a possibly modulated temperature gradient decreasing overall downwards by 0.2 to 2°C/hour, preferably 0.5 to 2°C/hour and still more preferably from 0.5 to 1.2°C/hour of treatment,
– that said crystallization zone is supplied in the vicinity of its upper end, on the one hand, with maltitol syrup having a richness in maltitol higher than 80% by weight and a proportion of dry matter higher than 65% and, on the other hand, with mass subject to crystallization which is taken up and recycled from an intermediate level of the crystallization zone, spaced from its ends by at least 1/6, preferably 1/5 and still more preferably 1/3 of the total length of said zone, the amount of mass subject to crystallization and recycled representing by volume from 40 to 110% of the amount of maltitol syrup introduced into the zone, and
– that there is extracted, continuously, in the vicinity of the lower end of the crystallization zone, a product highly enriched in maltitol crystals from which said crystals are recovered.

2. Method according to claim 1, characterized by the fact that the overall decreasing temperature gradient is from 0.5 to 1.2°C/hour treatment.

3. Method according to one of claims 1 and 2, characterized by the fact that the syrup serving as raw material has a richness in maltitol of 88 to 98%, preferably of 90 to 95% by weight and a proportion of dry matter of 70 to 87% by weight.

4. Method according to one of claims 1 to 3, characterized by the fact that the recycled fraction represents by volume from 80 to 100% of the volume of maltitol syrup supplying the crystallization zone.

5. Method according to claim 1, characterized by the fact that the temperature of the maltitol syrup at the moment of its introduction into the crystallization zone is from 50 to 90°C, preferably from 55 to 80°C and still more preferably from 60 to 70°C and that the mass extracted from the crystallization zone is, at the outlet of the latter, at a temperature from 40 to 20°C, preferably from 30 to 25°C.

6. Installation for the production of crystallized maltitol characterized by the fact that it is comprised essentially of a crystallization vessel of vertical or inclined axis and equipped
   – with a system of supply in syrup rich in maltitol in the vicinity of its upper end,
   – with a system of malaxation and a system of temperature regulation adapted to establish inside the vessel and within the mass subject to crystallization contained in the vessel, a temperature gradient decreasing overall from top to bottom,
   – with a system of continuous extraction in the vicinity of its lower end, of a product highly enriched in maltitol crystals which is conducted by suitable means to a system adapted to recover the crystals from this product, and
   – with means adapted to take up at an intermediate level of the vessel, spaced from the ends of the latter by at least 1/6, preferably 1/5 and still more preferably 1/3 of its total length, a fraction of the mass subjected to crystallization and to recycle this fraction into the vessel at a level close to the upper end of the latter.

EP 0 202 165 B1